# EUROPEAN PATENT APPLICATION

(11) **EP 3 176 259 A1**
(43) Date of publication of application: **07.06.2017**
(21) Application number: 15827983.6
(22) Date of filing: 28.07.2015
(51) Int. Cl.: C12N 9/02, C02F 1/00, C02F 3/34

(54) **METHOD FOR DECOLORIZING DYE**

(30) Priority: 28.07.2014 JP 2014153326
(71) Applicant: National University Corporation Chiba University, Chiba-shi, Chiba 263-0022 (JP); Godo Shusei Co., Ltd., Tokyo 104-8162 (JP)
(72) Inventor: AMACHI, Seigo, Matsudo-shi Chiba 271-8510 (JP); YOSHIKAWA, Jun, Matsudo-shi Chiba 271-0064 (JP); HORIGUCHI, Hirofumi, Matsudo-shi Chiba 271-0064 (JP)
(74) Representative: Schiener, Jens
(86) International application number: PCT/JP2015/071375
(87) International publication number: WO 2016/017640

(57) **Abstract**

A novel composition for decolorizing a dye is provided.

A composition for decolorizing a dye contains a multi copper oxidase and an iodide ion.

## Description

### Technical Field

The present invention relates to a method for decolorizing various dyes and a composition used therefor.

### Background Art

Mostofvariousprintedmatters, fibers, and other products have been colored using a dye. In order to recycle or dispose of these products, it is necessary to decolorize dyes thereof. In addition, decolorization is necessary in a wastewater treatment in a factory, or the like.

As a method for decolorizing such a dye, decolorization using bacteria in activated sludge (Patent Literature 1), decolorization by a peroxidase derived from Geotrichs candidum (Patent Literature 2), decolorization by hydrogen peroxide and a peroxidase (Patent Literature 3), and decolorization by Klebsiella Flavobacterium and Aeromonas bacteria (Patent Literature 4) have been reported.

### Citation List

### Patent Literature

Patent Literature 1: JP H10-323185 A
Patent Literature 2: JP 2000-245468 A
Patent Literature 3: JP 2001-9466 A
Patent Literature 4: JP 2002-28691 A

### Summary of Invention

### Technical Problem

However, a conventional decolorization means has the following problems. For example, a decolorization effect is not sufficient, a strong bleaching agent such as hydrogen peroxide is used, a treatment is performed under a lowpH condition, and the like.

Therefore, an obj ect of the present invention is to provide a novel decolorization means capable of decolorizing a dye safely.

### Solution to Problem

Therefore, the present inventors have focused on and studied a multicopper oxidase produced by a microorganism. As a result, the present inventors have found that a dye can be decolorized strongly by action of combination of the multicopper oxidase and an iodide ion on the dye, whereas the multicopper oxidase alone does not act on the dye, and have completed the present invention.

That is, the present invention provides the following [1] to [12].

[1] A composition for decolorizing a dye, containing a multicopper oxidase and an iodide ion.
[2] The composition for decolorizing a dye described in [1], in which the multicopper oxidase is a laccase.
[3] The composition for decolorizing a dye described in [1] or [2], in which the multicopper oxidase is a laccase derived from alpha-proteobacteria.
[4] The composition for decolorizing a dye described in any one of [1] to [3], in which the dye is an organic dye having a chromophore selected from the group consisting of >C=C<, >C=O, >C=N-, -N=N-, -N-O-, and -NO₂.
[5] A method for decolorizing a dye, characterized in that a composition containing a multicopper oxidase and an iodide ion acts on a dye.
[6] The method for decolorizing a dye described in [5], in which the multicopper oxidase is a laccase.
[7] The method for decolorizing a dye described in [5] or [6], in which the multicopper oxidase is a laccase derived from alpha-proteobacteria.
[8] The method for decolorizing a dye described in any one of [5] to [7], in which the dye is an organic dye having a chromophore selected from the group consisting of >C=C<, >C=O, >C=N-, -N=N-, -N-O-, and -NO₂.
[9] A decolorization composition containing a multicopper oxidase, an iodide ion, and a dye.
[10] The decolorization composition described in [9], in which the multicopper oxidase is a laccase.
[11] The decolorization composition described in [9] or [10], in which the multicopper oxidase is a laccase derived from alpha-proteobacteria.
[12] The decolorization composition described in any one of [9] to [11], in which the dye is an organic dye having a chromophore selected from >C=C<, >C=O, >C=N-, -N=N-, -N-O-, and -NO₂.

### Advantageous Effects of Invention

By use of a composition for decolorizing a dye according to the present invention, various colored compositions including wastewater can be decolorized.

### Brief Description of Drawings

Fig. 1 illustrates an optimum pH for ABTS. C. versicolor indicates a multicopper oxidase (MCO) derived from coriolus versicolor. IOE indicates MCO derived from a Q-1 strain.
Fig. 2 illustrates an optimum pH for 2,6-DMP. C. versicolor indicates a multicopper oxidase (MCO) derived from coriolus versicolor. IOE indicates MCO derived from a Q-1 strain.
Fig. 3 illustrates an optimum pH for hydroquinone. C. versicolor indicates a multicopper oxidase (MCO) derived from coriolus versicolor. IOE indicates MCO derived from a Q-1 strain.
Fig. 4 illustrates an optimum pH for p-phenylenediamine. C. versicolor indicates a multicopper oxidase (MCO) derived from coriolus versicolor. IOE indicates MCO derived from a Q-1 strain.
Fig. 5 illustrates temperature stability for p-phenylenediamine oxidation. C. versicolor indicates a multicopper oxidase (MCO) derived from coriolus versicolor. IOE indicates MCO derived from a Q-1 strain.
Fig. 6 illustrates temperature stability for ABTS oxidation. C. versicolor indicates a multicopper oxidase (MCO) derived from coriolus versicolor. IOE indicates MCO derived from a Q-1 strain.
Fig. 7 illustrates an optimum temperature for p-phenylenediamine oxidation. C. versicolor indicates a multicopper oxidase (MCO) derived from coriolus versicolor. IOE indicates MCO derived from a Q-1 strain.
Fig. 8 illustrates an optimum temperature for ABTS oxidation. C. versicolor indicates a multicopper oxidase (MCO) derived from coriolus versicolor. IOE indicates MCO derived from a Q-1 strain.
Fig. 9 illustrates a decolorization ability of each enzyme for RBBR (KI 1 mM, RBBR 0.3 mM, 0.01 U/mL as ability for ABTS oxidation). IOE: MCO alone, derived from a Q-1 strain. IOE + KI: combination of MCO derived from a Q-1 strain and KI. Pleurotus ostreatus : MCO alone, derived frompleurotus ostreatus. Pleurotus ostreatus + KI: combination of MCO derived from pleurotus ostreatus and KI. Coriolus versicolor: MCO derived from coriolus versicolor. Coriolus versicolor + KI: combination of MCO derived from coriolus versicolor and KI. KI: KI alone. RBBR only: RBBR alone.
Fig. 10 illustrates decolorization ability for RBBR in the presence of a mediator (KI 1 mM, ABTS 10 µM, HBT 1 mM, RBBR 0.3 mM, 0.01 U/mL) . IOE + KI: combination of MCO derived from a Q-1 strain, and KI. IOE + ABTS: combination of MCO derived from a Q-1 strain and ABTS. corio + ABTS: combination of MCO derived from coriolus versicolor and ABTS. corio + HBT: combination of MCO derived from coriolus versicolor and HBT.
Fig. 11 illustrates an influence of a KI concentration on RBBR decolorization of MCO derived from a Q-1 strain (RBBR 0.3 mM, 3.3 × 10⁻³ U/mL, four hours).
Fig. 12 illustrates an effect of apH on RBBRdecolorization of MCO derived from a Q-1 strain (KI 1 mM, RBBR 0.3 mM, 3.3 × 10⁻³ U/mL, three hours).
Fig. 13 illustrates RBBR decolorization ability in the absence of a mediator (RBBR 0.3 mM, 0.01 U/mL). IOE: corio of MCO derived from a Q-1 strain: MCO derived from coriolus versicolor.
Fig. 14 illustrates decolorization of orange G by MCO derived from a Q-1 strain (KI 1 mM, orange G 0.3 mM, 3.3 × 10⁻³ U/mL). KI + IOE: combination of MCO derived from a Q-1 strain and KI. KI: KI alone. IOE: MCO derived from a Q-1 strain alone.
Fig. 15 illustrates an effect of a pH on decolorization of orange G by MCO derived from a Q-1 strain (KI 1 mM, orange G 0.3 mM, 3.3 × 10⁻³ U/mL, three hours). +KI: KI is added, -KI: KI is not added.
Fig. 16 illustrates decolorization of methyl red by MCO derived from a Q-1 strain (KI 1 mM, methyl red 0.3 mM, 3.3 × 10⁻³ U/mL). KI + IOE : combination of MCO derived from a Q-1 strain and KI. KI: KI alone. IOE : MCO alone, derived from a Q-1 strain.
Fig. 17 illustrates decolorization of amide black by MCO derived from a Q-1 strain (KI 1 mM, amide black 0.3 mM, 3.3 × 10⁻³ U/mL). KI + IOE: combination of MCO derived from a Q-1 strain and KI. IOE: MCO alone, derived from a Q-1 strain.

### Description of Embodiments

A composition for decolorizing a dye according to the present invention contains a multicopper oxidase and an iodide ion.

The multicopper oxidase is an enzyme containing four copper atoms necessary for an enzymatic activity in a molecule thereof, is an enzyme which performs four-electron reduction for oxygen and generates a water molecule, and includes a laccase and a bilirubin oxidase. Among these enzymes, a laccase is preferable.

The laccase is an oxidase for oxidizing a phenol, and is present in plants, fungi, bacteria, and animals, for example. Among these laccases, a laccase derived from fungi or bacteria is preferable. Examples of fungi and bacteria for generating a laccase include alpha-proteobacteria, aspergillus, neurospora, podospora, botrytis, collybia, fomes, lentinus, pleurotus, trametes, rhizoctonia, coprinus, psathyrella, miceliophthora, scytalidium, polypolus, phlebia, coriolus, bacillus, pseudomonas, and escherichia. However, among these examples, alpha-proteobacteria is preferable.

A laccase used in the present invention is more preferably a laccase derived from alpha-proteobacteria. Examples of the laccase derived from alpha-proteobacteria include a laccase derived from a Q-1 strain, which has been found by the present inventors (Microbial Ecology, Vol. 49, 547-557 (2005), Applied And Environmental Microbiology, Vol. 78, No.11, 3941-3949 (2012)). Here, the Q-1 strain (accession number NITE BP-01864: deposited with National Institute of Technology and Evaluation Patent Microorganisms Depositary Center at 2-5-8-112, Kazusakamatari, Kisarazu, Chiba, Japan on June 3, 2014) is a new genus and new species bacterium belonging to a Kordiimonadales order. The present inventors have reported that the Q-1 strain generates amulticopper oxidase for oxidizing an iodide ion in the above literature. However, it is not known at all that combination of the multicopper oxidase generated by the Q-1 strain and an iodide ion decolorizes a dye.

A multicopper oxidase used in the present invention preferably has such a property that a reaction pH is from 5 to 8 and the multicopper oxidase is stable (maintains a specific activity of 60% or more) at from 20 to 60°C.

The composition for decolorizing a dye according to the present invention contains a multicopper oxidase. The composition may contain the multicopper oxidase as an enzyme separated or as a culture solution of a multicopper oxidase-producing bacterium containing a multicopper oxidase. In addition, the multicopper oxidase may be produced in the composition containing the multicopper oxidase-producing bacterium.

The content of the multicopper oxidase in the composition for decolorizing a dye according to the present invention is preferably from 0.0001 to 1000 U/mL, more preferably from 0.001 to 100 U/mL, and still more preferably from 0.01 to 10 U/mL as a concentration at the time for acting on a dye from a viewpoint of an effect for decolorizing a dye. When the multicopper oxidase-producing bacterium is used, the multicopper oxidase-producing bacterium only needs to be added such that the multicopper oxidase has a concentration within this range at the time for acting on a dye.

The content of the multicopper oxidase itself in the composition for decolorizing a dye depends on a specific activity of an enzyme and a dilution ratio at the time of use, for example. However, the content is preferably from 0.001 to 80% by mass, more preferably from 0.1 to 50% by mass, and still more preferably from 0.1 to 20% by mass. In addition, when the multicopper oxidase-producing bacterium is used, the content thereof is preferably from 0.01 to 80% by mass, more preferably from 0.1 to 50% by mass, and still more preferably from 0.1 to 20% by mass.

The multicopper oxidase alone exhibits no effect for decolorizing a dye. Therefore, the composition for decolorizing a dye according to the present invention needs to contain an iodide ion in addition to the multicopper oxidase. The iodide ion only needs to be generated at the time for acting on a dye. Therefore, the iodide ion is preferably added in a form of an alkali metal iodide such as potassium iodide or sodium iodide. As a source of the iodide ion, a generally commercially available reagent may be used. However, iodide ion-rich groundwater in nature, such as natural gas salt water, may be used.

The content of the iodide ion in the composition for decolorizing a dye is preferably from 0.001 to 1000 mM, more preferably from 0.01 to 100 mM, and still more preferably from 0.1 to 10 mM as a concentration at the time for acting on a dye from a viewpoint of an effect for decolorizing a dye.

The content of the iodide ion itself in the composition for decolorizing a dye depends on a dilution ratio at the time of use, for example. However, the content of an iodide is preferably from 0.001 to 80% by mass, more preferably from 0.01 to 50% by mass, and still more preferably from 0.1 to 20% by mass.

In addition to the above components, water, a pH adjusting agent, an excipient, an enzyme stabilizer, andasalt (for example, copper(II) chloride, copper(II) sulfate, sodium chloride, magnesium chloride, sodium sulfate, calcium chloride, potassium chloride, sodium bicarbonate, potassium bromide, strontium chloride, boric acid, sodium silicate, sodium fluoride, ammonium nitrate, sodium phosphate, or iron citrate) can be blended with the composition for decolorizing a dye according to the present invention.

The content of a copper ion in the composition for decolorizing a dye is preferably from 0.5 µM to 50 mM, more preferably from 5 µM to 5 mM, and still more preferably from 50 to 500 µM as a concentration at the time for acting on a dye from a viewpoint of an effect for decolorizing a dye.

Examples of a dye which can be decolorized by the composition for decolorizing a dye according to the present invention include an organic dye. Preferable examples thereof include a dye having a chromophore such as >C=C<, >C=O,>C=N-, -N=N-, -N=O-, -N-O-, or -NO₂. A dye having -CH₃-, -CN, -COOH, -O-, -OH, -OR, -NH₂, -NR², -Cl, -Br, -NO₂, or -SO₃H (R is an alkyl group) as an auxochrome is more preferable. More specific examples thereof include an azoic dye, an azo dye, an aniline dye, an anthraquinone dye, alizin, indanthrene, eosin, mango red, dihydroindole, methylene blue, a phenazine derivative dye, phenolphthalein, fuchsin, fluorescein, para red, mauve, flavonoid, a quinone dye, a porphyrin dye, a phycopilin dye, and an indigo dye.

When the composition containing a multicopper oxidase and an iodide ion dye acts on a dye, the dye can be decolorized. The compositions used here is similar to the above composition for decolorizing a dye. Therefore, examples of a combination of components for acting on a dye include a combination of a multicopper oxidase and an iodide ion, a combination of a multicopper oxidase-producing bacterium and an iodide ion, and a combination of a multicopper oxidase-producing bacterium culture and an iodide ion.

Conditions for acting on a dye only need to cause a multicopper oxidase to exhibit an activity. However, for example, an action under conditions of from 10 to 90°C and pH 3 to 10 for 0.01 to 240 hours is preferable. An action under conditions of from 20 to 80°C and pH 4 to 9 for 0.1 to 24 hours is more preferable. The concentration of a multicopper oxidase at the time for acting on a dye is preferably from 0.0001 to 1000 U/mL, more preferably from 0.001 to 100 U/mL, and still more preferably from 0.01 to 10 U/mL. The concentration of an iodide ion at the time for acting on a dye is preferably from 0.001 to 1000 mM, more preferably from 0.01 to 100 mM, and still more preferably from 0.1 to 10 mM.

By performing the method for decolorizing a dye according to the present invention, a decolorized composition containing a multicopper oxidase, an iodide ion, and a dye is produced.

According to the present invention, a dye in wastewater containing an organic dye, paper in papermaking, a colored fiber, or a coloring agent for hairs, for example, can be decolorized safely.

### [Examples]

Hereinafter, the present invention will be described in more detail based on Examples, but the present invention is not limited in any way thereto.

### Reference Example 1

A Q-1 strain (accession number NITE BP-01864) described in Microbial Ecology, Vol. 49, 547-557 (2005) and Applied And Environmental Microbiology, Vol. 78, No.11, 3941-3949 (2012) is a new genus and new species bacterium belonging to a Kordiimonadales order. A proposal to name the bacterium Iodidimonas marina has been made.

An enzyme to catalyze an iodine oxidation reaction of the Q-1 strain is an extracellular secreted enzyme, and requires not hydrogen peroxide but oxygen. Therefore, the enzyme has been considered to be not a known haloperoxidase but an oxidase-like enzyme. Furthermore, in the presence of a copper ion, the enzyme production amount of the Q-1 strain was increased by about 20 times, and the enzyme itself contained copper as a cofactor. An enzyme purified from a culture supernatant exhibited an oxidation activity to a methoxy phenol, a p-diphenol, and an aromatic diamine, such as 2,2'-azinobis(3-ethylbenzothiaziline-6-ammonium sulfonate (ABTS), p-phenylenediamine, syricagaldazine, 2,6-dimethoxyphenol (2,6-DMP), or hydroquinone, in addition to an iodide ion. In addition, the enzyme had maximum absorption derived from type 3 and type 1 copper at 320 nm and 590 nm, respectively. The above results strongly suggested that the enzyme for oxidizing iodine was a multicopper oxidase (MCO).

In order to estimate a structural gene of the enzyme, the Q-1 strain was subjected to draft genome analysis by Illumina GA II. An internal amino acid sequence obtained by subjecting a purified sample of the enzyme to a trypsin treatment and peptide fragment information obtainedbyLC-MS/MS analysis were compared with a draft genome sequence of the Q-1 strain. As a result, it was suggested that two ORFs (ioxA, ioxC) in the genome encoded the enzyme for oxidizing iodine. As a result of BLASTP analysis, IoxA exhibited homology with an estimated MCO of Roseovarius sp. 217 systematically close to a bacterium for oxidizing iodine in group 1, and IoxC exhibited homology with an unknown functional protein of the Roseovarius sp. 217. As a result of molecular phylogenetic analysis, it has been clear that IoxA is a novel MCO systematically different from a known MCO (for example, CopA, CotA, or CueO) derived from a bacterium (the above literature).

### Example 1

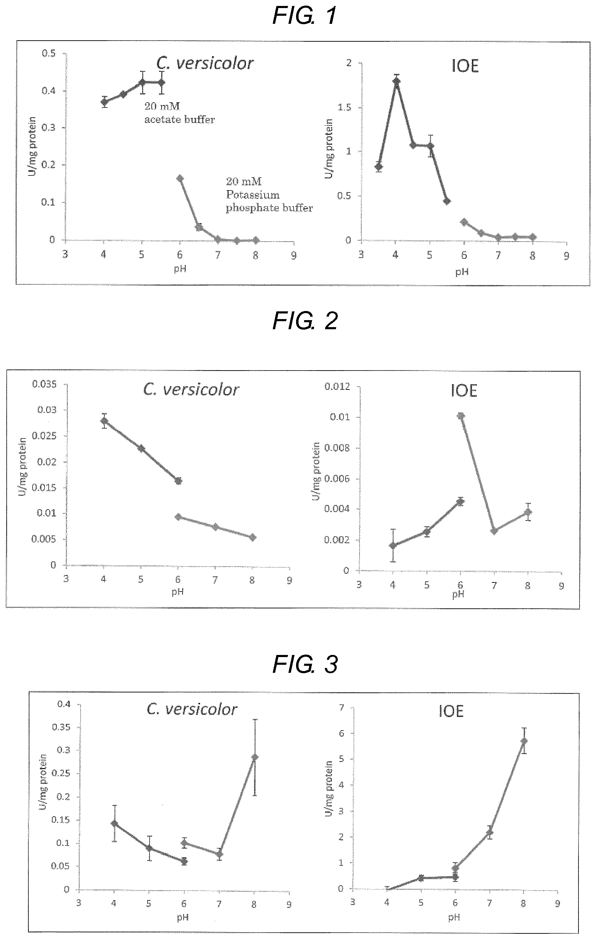

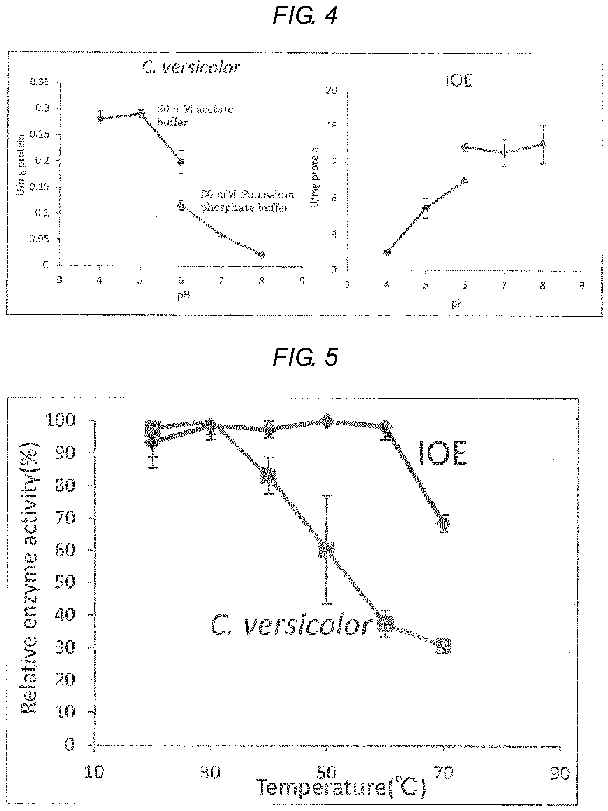

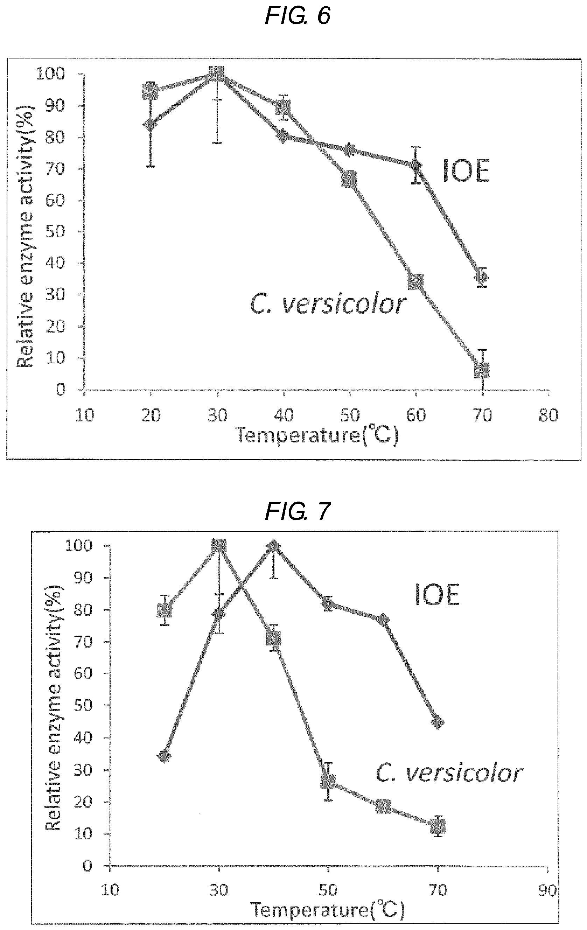

A specific activity, Km, Kcat, a reaction pH, and a reaction temperature were examined for an MCO derived from a filamentous fungus and an MCO derived from Q-1. As a substrate, ABTS, 2, 6-DMP, hydroquinone, p-phenylenediamine (pPD), and syringaldazine (SGZ) were used. The MCO derived from Q-1 was cultured in a medium obtained by adding a copper (II) chloride aqueous solution to Marine, Broth 2216 manufactured by Becton Dickinson Co., Ltd. so as to obtain a copper ion concentration of 40 µM (final concentration) at 30°C for two days. A culture supernatant of the resulting culture solution was used as a crude enzyme. As the MCO derived from a filamentous fungus, an MCO derived from coriolus versicolor (brand name "Fluka" manufactured by Sigma-Aldrich Co. LLC.; product name "Laccase, Coriolus versicolor, CLEA"; product number "38837") was used. Tables 1 and 2 and Figs. 1 to 8 illustrate results thereof.

Figs. 1 to 8 illustrate that an optimum pH of the MCO derived from a filamentous fungus was from 3 to 5, while an optimum pH of the MCO derived from a Q-1 strain was from 4 to 8, particularly from 6 to 8 in a neutral region. In addition, as temperature stability of the Q-1 strain MCO, the Q-1 strain MCO was stable in a wide range of 20 to 60°C.

**[Table 1]**

| Activity of Coriolus versicolor against each substrate | | | | | | |
|---|---|---|---|---|---|---|
| | ABTS | 2,6-DMP | Hydroquinone | pPD | SGZ | KI |
| specific activity (U/mg protein) | 2.15×10⁻¹ | 2.65×10⁻² | 2.42×10⁻³ | 1.11×10⁻¹ | 4.17×10⁻⁴ | N.D. |
| Kₘ (mM) | 6.16×10⁻² | 5.19×10⁻² | 1.34×10⁻² | 9.21×10⁻² | 3.79 | N.D. |
| k_{cta} (min⁻¹) | 2.10×10 | 3.83 | 2.71×10⁻¹ | 6.20 | 6.82×10² | N.D. |
| k_{cat}/Kₘ (min⁻¹M⁻¹) | 3.40×10⁵ | 7.38×10⁴ | 2.03×10⁴ | 6.73×10⁴ | 1.80×10 | N.D. |

**[Table 2]**

| Activity of IOE against each substrate | | | | | | |
|---|---|---|---|---|---|---|
| | ABTS | 2,6-DMP | Hydroquinone | pPD | SGZ | KI |
| specific activity (U/mg protein) | 1.22 | 3.4×10⁻³ | 3.57×10⁻¹ | 1.68×10 | N.D. | 2.49×10 |
| Kₘ (mM) | 2.66×10⁻² | 4.78×10⁻² | 2.31 | 7.65×10⁻¹ | N.D. | 2.42×10 |
| k_{cta} (min⁻¹) | 2.35×10² | 4.87×10 | 7.20×10² | 4.67×10³ | N.D. | 2.22×10⁴ |
| k_{cat}/Kₘ (min⁻¹M⁻¹) | 8.83×10⁶ | 1.02×10⁴ | 2.33×10⁵ | 6.11×10⁶ | N.D. | 9.19×10⁵ |

### Example 2

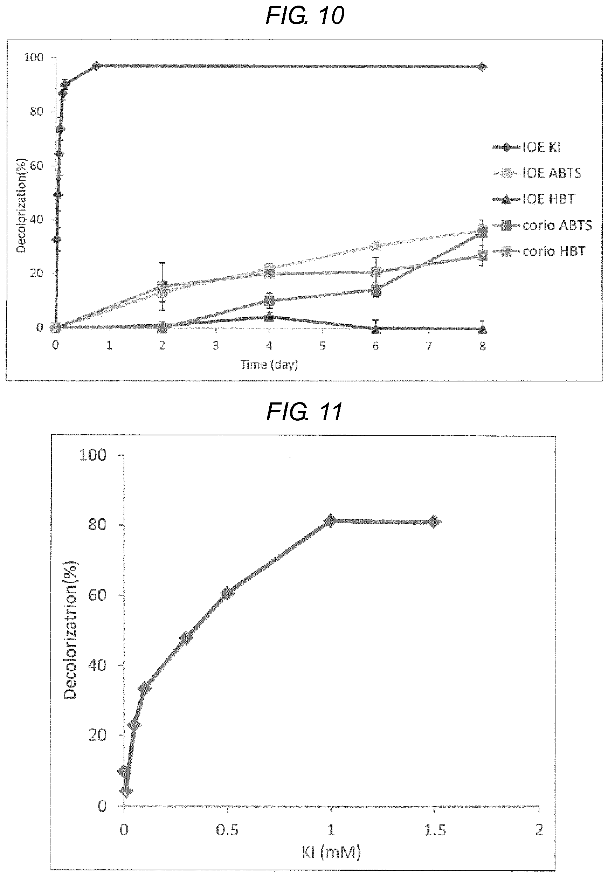

A dye decolorization ability of the MCO derived from a Q-1 strain was examined. Examination was performed in the presence or the absence of KI. As a dye, RBBR, methyl red, orange G, and amide black were used. Reaction conditions were pH 7, 30°C, and from 5 to 18 hours (Fig. 10 illustrates a case of 8 days).

A 20 mM acetic acid buffer solution was added to each of the MCO derived from coriolus versicolor used in Example 1 and an MCO derived from pleurotus ostreatus (product name Laccase from Pleurotus ostreatus (mushroom) manufactured by Sigma-Aldrich Co. LLC.) as the MCO derived from a filamentous fungus to obtain a pH of 4.0. Other reaction conditions were 30°C and from 5 to 18 hours (Fig. 10 illustrates a case of 8 days).

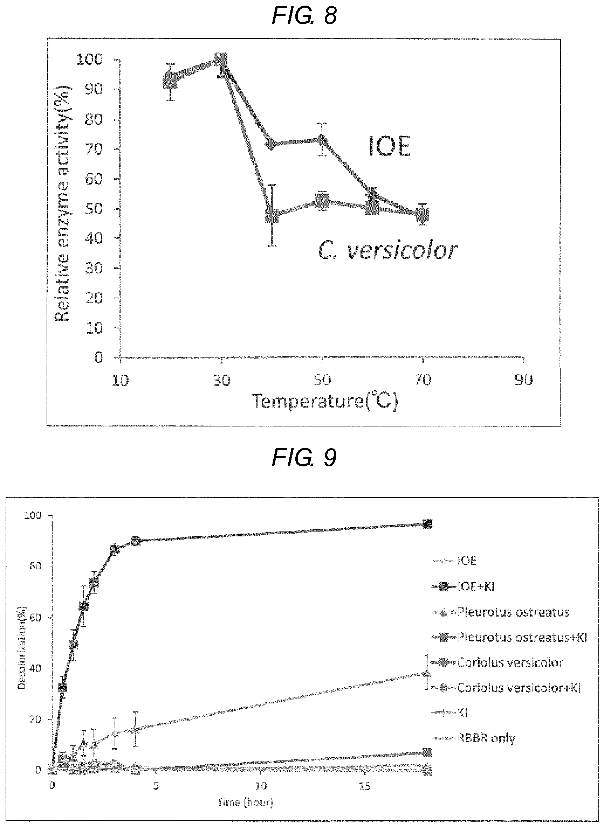

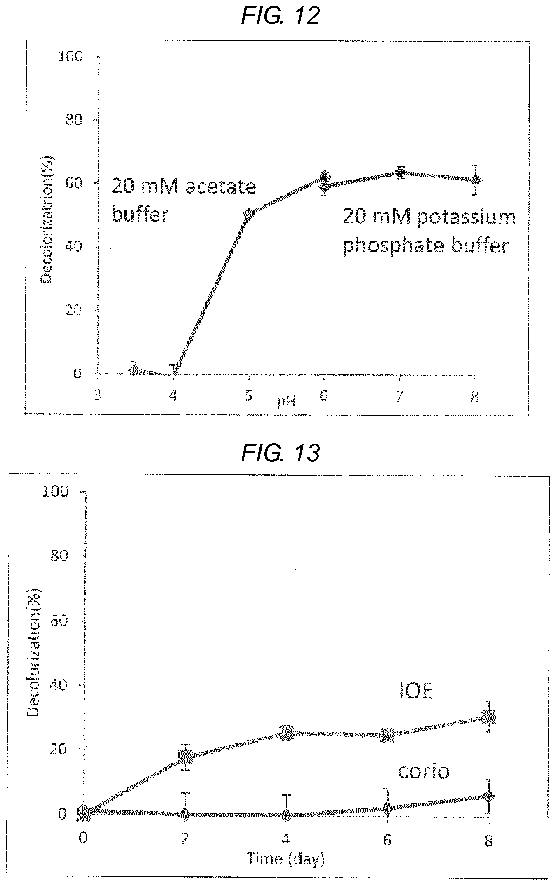

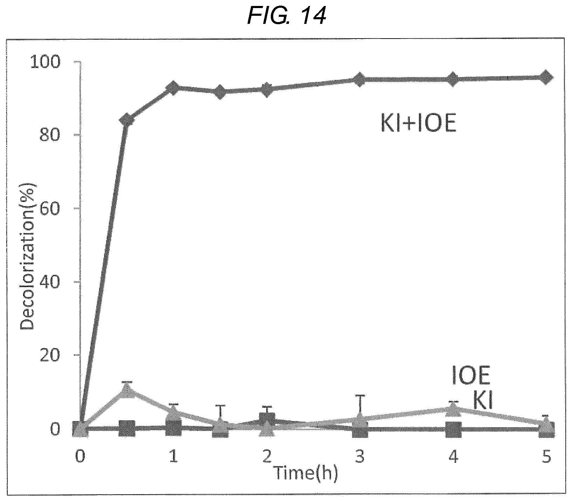

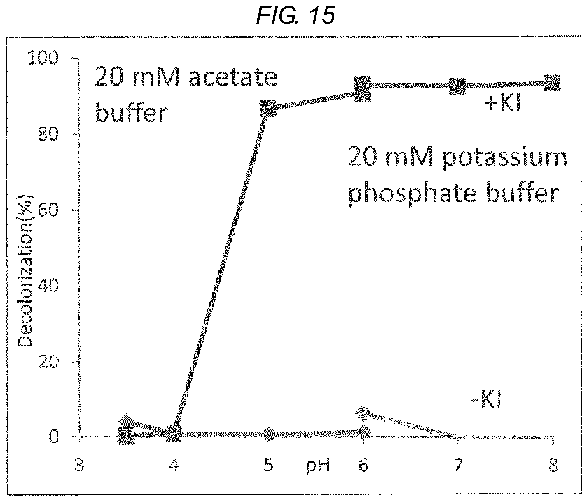

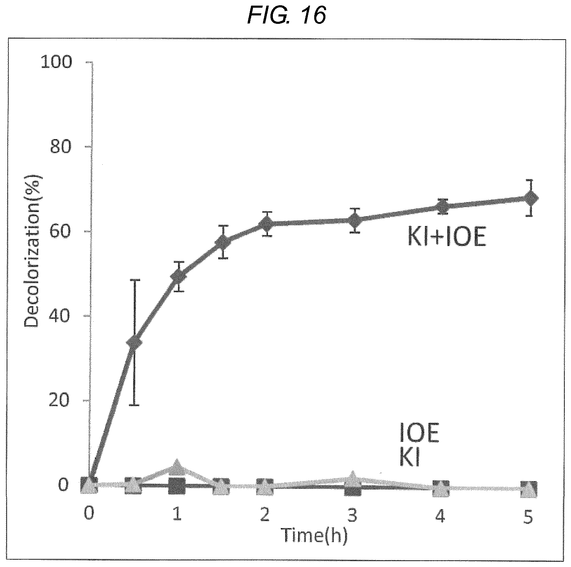

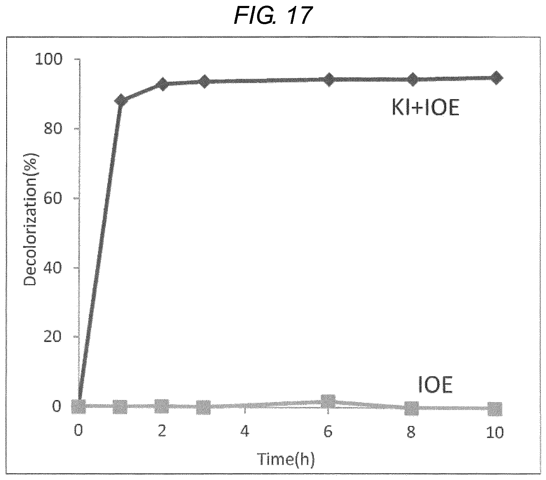

Figs. 9 to 17 illustrate results thereof. As clear from Figs. 9 to 17, the Q-1 strain MCO alone did not exhibit a dye decolorization ability, but exhibited a strong dye decolorization ability in the presence of an iodide ion.

## Claims

1. A composition for decolorizing a dye, comprising a multicopper oxidase and an iodide ion.

2. The composition for decolorizing a dye according to claim 1, wherein the multicopper oxidase is a laccase.

3. The composition for decolorizing a dye according to claim 1 or 2, wherein the multicopper oxidase is a laccase derived from alpha-proteobacteria.

4. The composition for decolorizing a dye according to any one of claims 1 to 3, wherein the dye is an organic dye having a chromophore selected from the group consisting of >C=C<, >C=O, >C=N-, -N=N-, -N-O-, and -NO₂.

5. A method for decolorizing a dye, wherein a composition containing a multicopper oxidase and an iodide ion acts on a dye.

6. The method for decolorizing a dye according to claim 5, wherein the multicopper oxidase is a laccase.

7. The method for decolorizing a dye according to claim 5 or 6, wherein the multicopper oxidase is a laccase derived from alpha-proteobacteria.

8. The method for decolorizing a dye according to any one of claims 5 to 7, wherein the dye is an organic dye having a chromophore selected from the group consisting of >C=C<, >C=O, >C=N-, -N=N-, -N-O-, and -NO₂.

9. A decolorization composition comprising a multicopper oxidase, an iodide ion, and a dye.

10. The decolorization composition according to claim 9, wherein the multicopper oxidase is a laccase.

11. The decolorization composition according to claim 9 or 10, wherein the multicopper oxidase is a laccase derived from alpha-proteobacteria.

12. The decolorization composition according to any one of claims 9 to 11, wherein the dye is an organic dye having a chromophore selected from the group consisting of >C=C<, >C=O, >C=N-, -N=N-, -N-O-, and -NO₂.
